(19) Europäisches Patentamt
European Patent Office
Office européen des brevets

(11) Veröffentlichungsnummer: **0 029 488**
**A1**

# (12) EUROPÄISCHE PATENTANMELDUNG

(21) Anmeldenummer: 80105514.6

(22) Anmeldetag: 13.09.80

(51) Int. Cl.³: **C 07 D 209/42**
C 07 D 215/48, C 07 D 217/26
C 07 D 401/12, C 07 D 401/14
A 61 K 31/40, A 61 K 31/47

(30) Priorität: 19.09.79 DE 2937779
21.11.79 DE 2946909

(43) Veröffentlichungstag der Anmeldung:
03.06.81 Patentblatt 81/22

(84) Benannte Vertragsstaaten:
AT BE CH DE FR GB IT LI NL SE

(71) Anmelder: HOECHST AKTIENGESELLSCHAFT
Zentrale Patentabteilung Postfach 80 03 20
D-6230 Frankfurt/Main 80(DE)

(72) Erfinder: Geiger, Rolf, Prof. Dr.
Heinrich-Bleicher-Strasse 33
D-6000 Frankfurt am Main 50(DE)

(72) Erfinder: Teetz, Volker, Dr.
An der Tann 20
D-6238 Hofheim am Taunus(DE)

(72) Erfinder: Schölkens, Bernward, Dr.
Am Fliedergarten 1
D-6233 Kelkheim (Taunus)(DE)

(72) Erfinder: Wissmann, Hans, Dr.
Falkenstrasse 12
D-6232 Bad Soden am Taunus(DE)

(54) **Aminosäurederivate, Verfahren zu ihrer Herstellung und ihre Verwendung zur Behandlung von Bluthochdruck.**

(57) Aminosäurederivate der Formel I

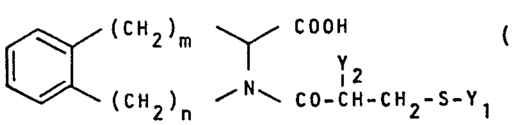

(I)

worin m, n, $Y_1$ und $Y_2$ die angegebene Bedeutung haben, sowie deren Salze und Verfahren zu ihrer Herstellung. Die Verbindungen besitzen wertvolle pharmakologische Eigenschaften.

EP 0 029 488 A1

Croydon Printing Company Ltd.

BEZEICHNUNG GEÄNDERT
siehe Titelseite



HOECHST AKTIENGESELLSCHAFT HOE 79/F 250 K Dr.LI/mh

## Aminosäurederivate und Verfahren zu ihrer Herstellung

Gegenstand der Erfindung sind Verbindungen der Formel

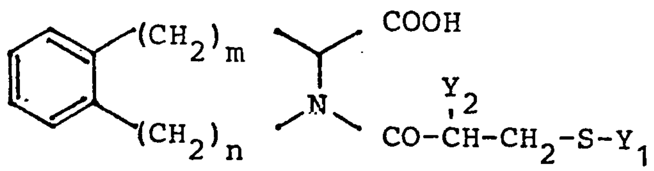

(I)

in welcher bedeutet n 0 oder 1, m 1 oder 2, oder $n+m \leq 2$, $Y_1$ Wasserstoff, Niederalkanoyl oder -cycloalkanoyl, Benzoyl, Nicotinoyl oder einen Rest der Formel II, wobei vorzugsweise m und n jeweils in beiden Molekülhälften dieselbe Bedeutung haben,

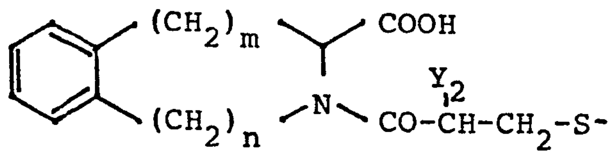

(II)

$Y_2$ Methyl, Amino, Niederalkanoylamido, Niedercycloalkanoylamido, Benzoylamido oder Nicotinoylamido.

Niederalkanoyl im Sinne des Vorstehenden bedeutet insbesondere Alkanoyl mit 1-6 C-Atomen, Niedercycloalkanoyl insbesondere Cycloalkanoyl mit 5-8 C-Atomen.

Die Verbindungen besitzen ähnlich wie viele andere Derivate des Indolins und des 1,2,3,4-Tetrahydroindolins wertvolle pharmakologische Eigenschaften.

Gegenstand der Erfindung ist ferner ein Verfahren zur Herstellung dieser Verbindungen. Dieses ist dadurch gekennzeichnet, daß man einen Niederalkylester einer Verbindung der Formel

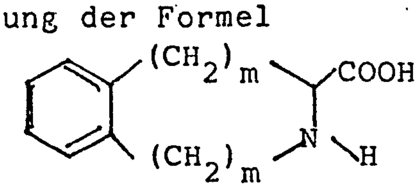

(III)

oder ein Salz dieser Verbindung mit einer starken, nicht flüchtigen organischen Base mit einem aktivierten Derivat einer Carbonsäure der Formel IV

$$HOOC-\underset{}{\overset{Y_2}{\overset{|}{C}}}H-CH_2-S-Y_1$$

in der $Y_1 \neq H$ ist, umsetzt, aus dem erhaltenen Produkt die organische Base bzw. die Estergruppe abspaltet, gegebenenfalls einen in der Position $Y_1$ stehenden Säurerest abspaltet oder durch einen anderen austauscht und, falls die Verbindung eine freie SH-Gruppe hat, gewünschtenfalls das Produkt zur Disulfidverbindung oxidiert.

Die Synthese der erfindungsgemäßen Verbindungen ist einfach. Die als Ausgangsprodukt in Frage kommende Indolin-2-carbonsäure ist aus Aust.J.Chem. 20 (1967), Seite 1935, die 1.2.3.3-Tetrahydrochinolin-2-carbonsäure aus Ber. 61 (1928), Seite 2377 bekannt. Die Thiolan-carbonsäuren der Formel IV kennt man aus Acta Chem. Scand. 9 (1955), Seite 178 und 10 (1956), Seite 678.

Die 1,2,3,4-Tetrahydroisochinolin-3-carbonsäure als Ausgangsmaterial ist durch Reaktion von Phenylanilin mit Formaldehyd nach J.Amer.Chem.Soc. 70 (948), Seite 182, leicht zugänglich. Die Carbonsäure der Formel IV

$$Y^1-S-CH_2-\underset{CH_3}{\underset{|}{C}}H-COOH$$

sind unter anderem in der DE-OS 2 752 720 beschrieben oder können analog hergestellt werden.

Die der Formel IV entsprechende Verbindung mit $Y_2$= Nicotinylamido wird aus Thinicotinsäure und Methacrylsäure hergestellt, wie sie ähnlich in der DE-OS 2 845 499 ausgeführt wird.

Geeignete Derivate des Cysteins sind literaturbekannt.

Die Herstellung der Säureamidbildung folgt den allgemeinen Verfahren, bevorzugt denen der Peptidchemie, wie sie in Houben-Weyl, Methoden der organischen Chemie, Band 15, ausführlich beschrieben sind.

Als aktivierte Derivate einer Carbonsäure der Formel IV kommen z.B. die Säurechloride, Aktivester, gemischte Anhydride mit beliebigen anderen Carbonsäuren oder Kohlensäurehalbestern in Frage. Sie lassen sich mit Salzen der Aminosäuren der Formel III, speziell dem tert.-Butylester oder mit Basensalzen, z.B. Tetraalkylguanidinium-, Trialkylbenzyl- oder Tetraalkylammoniumsalzen der 1,2,-3,4-Tetrahydroisochinolin-3-carbonsäure direkt umsetzt.

Aktivierte Ester werden z.B. aus der Säure und N-Hydroxysuccinimid, 2,4,5-Trichlorbenzol oder 1-Hydroxybenzotriazol in bekannter Weise mit Dicyclohexylcarbodiimid hergestellt und nach dem Abfiltrieren des bei der Herstellung entstehenden Dicyclohexylharnstoffs in Lösung sogleich verwendet. Bevorzugte Lösungsmittel sind Dimethylformamid, Dimethylacetamid, N-Methylpyrrolidon, Phosphorsäure-tris-dimethylamid oder Methylenchlorid. In denselben Lösungsmitteln werden die erfindungsgemäßen Salze der Aminosäuren der Formel III aufgenommen, die zuvor z.B. in alkoholischer oder wäßrig-alkoholischer Lösung aus den Komponenten in äquimolarer Menge erhalten und vom Lösungsmittel durch Abdestillieren im Vakuum befreit wurden.

Starke, nicht flüchtige organische Basen im Sinne der Erfindung sind insbesondere Tetraalkyl- oder-aralkylammoniumhydroxide oder tetraalkylierte Guanidine wie Tetramethylguanidin.

Verbindungen, in denen $Y_1$ Wasserstoff ist, können durch Behandeln der entsprechenden S-Acrylderivate mit Ammoniak oder Alkali- bzw. Erdalkalihydroxiden erhalten werden. Mit den letzten Agentien werden auch gleichzeitig evtl. vorhandene Methyl- oder Ethylester-Gruppen verseift.

Die Reaktion kann bei Raumtemperatur stattfinden und ist bei Verwendung der Säurechloride nach etwa 30 Minuten, bei Verwendung von 1-Hydroxybenzotriazolestern nach längstens 4 Stunden beendet. Man destilliert dann das Lösungsmittel im Vakuum ab und reinigt das Rohprodukt z.B. durch Flüssigkeitschromatographie an Silicagel, wobei sich Lösungsmittelsysteme mit Chloroform, Methanol und Essigsäure bewährten.

Man kann aber auch, wie das bevorzugt in der Peptidchemie geschieht, die Carbonsäuren IV mit Alkylestern der Aminosäuren der Formel III kondensieren und die Ester anschließend spalten oder verseifen, wobei im allgemeinen gleichzeitig die S-Acylbindung gespalten wird. Bevorzugtes Kondensationsmittel ist Dicyclohexyl-carbodiimid, gegebenenfalls unter Zusatz von N-Hydroxysuccinimid oder 1-Hydroxybenzotriazol. Bevorzugte Lösungsmittel sind die oben aufgeführten.

Verbindungen der Formel V

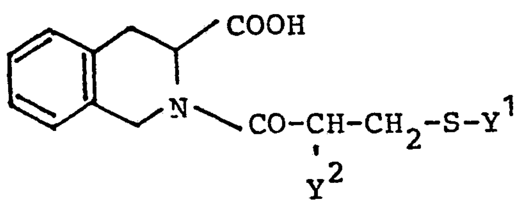

(V)

in der $Y_1$ Niederalkanoyl oder -cycloalkanoyl, Benzoyl oder Nicotinoyl und $Y_2$ Methyl oder Niederalkanoyl-, cycloalkanoyl-, Benzoyl- oder Nicotinoylamido ist, können erhalten

werden, indem man beispielweise einen Ester der Formel VI

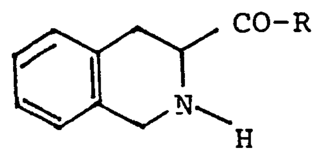

(VI)

in der R den Rest eines sauer abspaltbaren Alkohols, z.B den tert.-Butoxyrest darstellt, mit einer Carbonsäure der Formel IV umsetzt. In den so erhaltenen Verbindungen kann nach Säurespaltung der Estergruppierung -CO-R durch Umsetzung mit Ammoniak, einem primären oder sekundären Amin oder einem Alkalihydroxid und Behandlung des Salzes mit Säure oder einem stark sauren Ionenaustauscher der Rest $Y^1$ durch Wasserstoff ersetzt werden.

Man kann auch andere Alkylester, bevorzugt Methyl- oder Ethylester, der 1,2,3,4-Tetrahydro-isochinolin-3-carbonsäure mit einer Carbonsäure der Formel IV kondensieren und das Reaktionsprodukt zur Abspaltung der Methyl- oder Ethylester- und der S-Acylgruppe mit einem Alkali- oder Erdalkalihydroxid behandeln.

Ferner können Verbindungen der Formel V mit $Y_1$ = H und $Y_2$ = Amino dadurch hergestellt werden, daß man eine Verbindung der Formel VI mit N,S-Di-tert.-butyloxycarbonylcystein kondensiert und die Schutzgruppen durch Behandeln mit einer starken Säure, bevorzugt in Anwesenheit eines SH-Gruppen tragenden Kationenfängers wie Thiophenol oder Dithioglycol, abspaltet.
Weiterhin kann man ein Tetraalkylguanidinium-, Trialkylbenzyl- oder Tetraalkylammoniumsalz der 1,2,3,4-Tetrahydroisochinolin-3-carbonsäure mit einem aktivierten Derivat der Carbonsäure IV umsetzen und das erhaltene Salz mit Säure oder einem stark sauren Ionenaustauscher zerlegen.

Die erhaltenen Produkte können gegebenenfalls weiter modifiziert werden durch Abspaltung von Acylgruppen wie Acetyl oder Benzoyl in der Position $Y^1$, z.B. durch Behandeln mit Ammoniak, einem Alkali- oder Erdalkalihydroxid und gegebenenfalls Behandeln mit einem milden Oxidationsmittel wie Luft, Jod oder Kaliumhexacyanoferrat-(III) zwecks Einführung eines Restes der Formel II.

Zur Herstellung von Verbindungen der Formel I, in der $Y^1$ einen Rest der Formel II darstellt, kann man beispielsweise auch die 1,2,3,4-Tetrahydro-isochinolin-3-carbonsäure, ihre Salze oder Ester mit Verbindungen der Formel VII

$$HOOC-\overset{Y_2}{\overset{|}{C}}H-CH_2-S-S-CH_2-\overset{Y_2}{\overset{|}{C}}H-COOH \qquad (VII)$$

umsetzen und evtl.vorhandenen Schutzgruppen gewünschtenfalls abspalten.

Verbindungen der Formel I, in denen $Y_1$ Nicotinoyl darstellt, können auch erhalten werden, indem man eine entsprechende Verbindung der Formel III mit einem aktivierten-Derivat der Nicotinsäure umsetzt.

Je nach dem Charakter der Ausgangsstoffe, insbesondere der Bedeutung der Substituenten $Y^1$ und $Y^2$, kann das eine oder andere der beschriebenen Verfahren in einzelnen Fällen eine gewünschte individuelle Verbindung nur in geringen-Ausbeuten liefern oder zu deren Synthese nicht geeignet sein. In solchen verhältnismäßig selten auftretenden Fällen macht es dem Fachmann keine Schwierigkeiten, das gewünschte Produkt auf einem anderen der beschriebenen Verfahrenswege zu synthetisieren.

Die erfindungsgemäßen Verbindungen besitzen 1 - 2 chirale Zentren. Sie liegen zunächst als Harze vor und lassen sich zum Teil durch längeres Behandeln mit Petrolether in feste Form überführen. Sie schmelzen meist unter Zersetzung und

besitzen einen unscharfen Schmelzpunkt, der zudem stark von der Heizdauer abhängt. Ihre Identität wird durch Elementaranalyse, UV-, IR- und NMR-Spektroskopie gesichert.

Durch Gegenstromverteilung ihrer Salze mit optisch aktiven Basen konnten stereochemisch einheitlichere Verbindungen mit höherer biologischer Aktivität erhalten werden, deren absolute Konfiguration noch unbekannt ist. In praxi kann die Anwendung der stark und besonders langwirksamen Verbindungen auch in Form des Stereoisomerengemisches erfolgen.

Die erfindungsgemäßen Verbindungen erniedrigen bei peroraler Gabe von 1 bis 20 mg pro kg und Tag an der hypertonen Ratte und am Hund den Blutdruck und sind auch am Menschen in demselben Dosierbereich wirksam. Die Dosis konnte im Tierversuch ohne schädliche Wirkung erhöht werden.

Die neuen Verbindungen werden zur Bekämpfung des Bluthochdrucks verschiedener Genese eingesetzt. Sie können für sich oder in Kombination mit anderen blutdrucksenkenden, gefäßerweiternden oder diuretisch wirksamen Verbindungen angewandt werden. Typische Vertreter dieser Wirkklasse sind z.B. in Ehrhardt-Ruschig, Arzneimittel, 2. Auflage, Weinheim 1972, beschrieben.

Die Erfindung wird durch folgende Beispiele weiter erläutert:

Beispiel 1

N-(2-Methyl-3-acetylmercaptopropionyl)-L-1.2.3.4.-tetrahydro-isochinolin-3-carbonsäure

A. N-Benzyloxycarbonyl-L-1.2.3.4.-tetrahydro-isochinolin-3-carbonsäure

35 g L-1-2-3-4-Tetrahydro-isochinolin-3-carbonsäure, hergestellt aus L-Phenylalanin durch Kondensation mit Formaldehyd nach J.Amer.Chem.Soc. 70 (1948), Seite 182, werden in 200 ml 1N NaOH suspendiert. Unter starkem Rühren gibt man bei 0-5°C 30.2 ml Benzyloxycarbonylchlorid und 232 ml 1N NaOH gleichzeitig langsam zu und rührt noch 2 Stunden. Dann wird zweimal mit Ether extrahiert, die wäßrige Lösung mit konzentrierter Salzsäure angesäuert und mit Essigester ausgeschüttelt. Die Essigesterphase wird mit Wasser gewaschen und über $Na_2SO_4$ getrocknet. Nach Abdestillieren des Lösungsmittels im Vakuum hinterbleibt ein Harz. Es wird in heißem Diisopropylether gelöst. Beim Abkühlen kristallisiert nach Anreiben die Verbindung aus. Ausbeute 50.4 g, Schmp. 139°-140° $[\alpha]_D$:+23.9° (c=1 in Methanol).

B. N-Benzyloxycarbonyl-L-1.2.3.4-tetrahydro-isochinolin-3-carbonsäure-tert.-butylester

31.1 g der nach A erhaltenen Verbindung werden in 250 ml Methylenchlorid gelöst. Man gibt 38.5 ml tert.-Butanol und 1 g 4-Dimethylaminopyridin zu und tropft unter Rühren bei 0°C die Lösung von 22 g Dicyclohexylcarbodiimid in 60 ml Methylenchlorid zu. Man rührt dann 15 Minuten bei 0°C und 5 h bei Raumtemperatur, filtriert und bringt das Filtrat im Vakuum zur Trockene. Der Rückstand wird wieder in Methylenchlorid aufgenommen, die Lösung nacheinander mit gesättigter Natriumhydrogencarbonatlösung und Kaliumhydrogensulfat-/Kaliumsulfatlösung ausgeschüttelt, mit Wasser neutral gewaschen und über $Na_2SO_4$ getrocknet. Nach dem Eindampfen hinterbleibt ein öliger Rückstand. Ausbeute 32,6 g.

C. L-1.2.3.4-Tetrahydro-isochinolin-3-carbonsäure-tert.-butylester-toluolsulfonat

30.5 g der nach B erhaltenen Verbindung werden in 300 ml Methanol gelöst und an Palladiummohr katalytisch hydriert, wobei am Autotitrator durch Zugabe von 1N Toluolsulfosäure in Methanol der pH-Wert bei 5 gehalten wird. Nach beendeter Reaktion wird der Katalysator abfiltriert und das Lösungsmittel abdestilliert. Der harzige Rückstand wird beim Verreiben mit Ether fest. Ausbeute 31.4 g. Die Verbindung ist chromatographisch nahezu rein vom Ausgangsmaterial durch einen niedrigeren $R_f$-Wert im DC unterschieden.

D. N-(2-Methyl-3-acetylmercaptopropionyl)-L-1.2.3.4-tetrahydro-isochinolin-carbonsäure-tert.-butylester

Man löst 20,3 g des nach C erhaltenen tert.-Butylestertosylats in 200 ml Methylenchlorid, gibt 8,3 g 2-Methyl-3-acetylmercapto-propionsäure, 6.4 ml N-Ethylmorpholin und unter Eiskühlung 11 g Dicyclohexylcarbodiimid, in 60 ml Methylenchlorid gelöst, zu. Man rührt über Nacht bei Raumtemperatur, filtriert, destilliert das Lösungsmittel im Vakuum ab und nimmt den Rückstand in Essigester auf. Die Lösung wird nacheinander mit $NaHCO_3$-Lösung, $KHSO_4/K_2SO_4$-Lösung und Wasser gewaschen, über $Na_2SO_4$ getrocknet und im Vakuum eingedampft. Man erhält 16,3 g Öl.

E. N-(2-Methyl-3-acetylmercaptopropionyl)-L-1.2.3.4-tetrahydro-isochinolin-3-carbonsäure

13 g der nach D erhaltenen Verbindung löst man in 70 ml Trifluoressigsäure, die nach 45 min. im Vakuum abdestilliert wird. Man verteilt den Rückstand zwischen 180 ml Ethylether und 90 ml gesättigter $NaHCO_3$-Lösung. Die Etherphase wird verworfen, die wäßrige Phase mit konzentrierter Salzsäure angesäuert und dreimal mit Essigester extrahiert. Die vereinigten Essigesterlösungen werden mit Wasser gewaschen und über $Na_2SO_4$ getrocknet. Man destilliert das Lösungsmittel ab, nimmt

den Rückstand in Ether auf und versetzt unter Rühren solange mit Cyclohexylamin, bis feuchtes pH-Papier einen Wert von 8 anzeigt. Nach Kühlen der entstandenen Suspension filtriert man das Cyclohexylammoniumsalz ab und wäscht es mit Ether. Nach dem Trocknen wird es aus Isopropanol und Acetonitril umkristallisiert. Schmp. 159-161°, $[\alpha]_D$: -11,5° (c=0,5 in Methanol). Ausbeute 4,8 g. Zur Gewinnung der freien Säure wird das Salz zwischen Wasser und Essigester/Ether suspendiert. Nach Zugabe von $KHSO_4$-Lösung bis pH 2 geht alles in Lösung. Man trennt die Phasen, wäscht die wäßrige Lösung mit etwas Essigester und die vereinigten, organischen Phasen mit wenig Wasser, trocknet diese über $Na_2SO_4$ und destilliert das Lösungsmittel im Vakuum ab. Der harzige Rückstand wird beim Verreiben mit Ether fest. Ausbeute 3,0 g. Elementaranalyse korrekt.

Beispiel 2

N-(2-Methyl-3-mercaptopropionyl)-L-1.2.3.4-tetrahydro-isochinolin-3-carbonsäure

1,3 g der nach Beispiel 1 E hergestellten Verbindung werden in 8 ml 5N Ammoniak in Methanol 2 Stunden unter Stickstoff aufbewahrt. Dann bringt man die Lösung im Vakuum zur Trockne. Der feste Rückstand wird in 10 ml 50 %igem, wäßrigem Methanol gelöst. Man filtriert über eine kleine Säule mit dem stark sauren Ionenaustauscher Lewatit S 100 und bringt das Eluat im Vakuum zur Trockne. Der harzige Rückstand wird mit Petrolether digeriert, wobei er fest wird. Nach Trocknen im Vakuum erhält man die Titelverbindung in einer Ausbeute von 0,85 g. Elementaranalyse korrekt.

Beispiel 3

N-D-Cysteinyl-L-1.2.3.4-tetrahydro-isochinolin-3-carbonsäure

A. N.S.-Di-Boc-D-cysteiny-L-1.2.3.4-tetrahydro-isochinolin-3-carbonsäure-tert.-butylester

4,36 g N,S,-Di-Boc-D-cystein-N-hydroxysuccinimidester,

hergestellt analog Liebig's Ann.Chem. 743 (1971), Seite 57, und 4,05 g L-1.2.3.4-Tetrahydro-isochinolin-3-carbonsäure-tert.-butylester-tosylat, hergestellt nach Beispiel 1, werden in 60 ml Dimethylformamid gelöst. Man gibt 1.28 ml N-Ethylmorpholin zu und rührt über Nacht bei Rumtemperatur. Dann destilliert man das Lösungsmittel im Vakuum ab, digeriert den harzigen Rückstand mit Wasser und trocknet im Vakuum. Ausbeute 4,49 g (81 %) einer festen Masse von unscharfem Schmelzpunkt.

B. D-Cysteinyl-L-1.2.3.4-tetrahydro-isochinolin-3-carbonsäure

4,4 g der nach A erhaltenen Verbindung werden 15 - 20 Minuten in 0,2N HCl im Ameisensäure, die 5 % Dithioglycol enthält, bei Raumtemperatur aufbeahrt. Man bringt im Vakuum zur Trockene und digeriert den Rückstand mit Ether. Das erhaltene feste Produkt ist etwas hygroskopisch. Ausbeute 1.44 g (56 %). Elementaranalyse nach Berücksichtigung von 3-5 % Wasser korrekt.

Beispiel 4

N-Acetyl-S-Benzoyl-D-cysteinyl-L-1.2.3.4-tetrahydroisochinolin-3-carbonsäure

Man setzt 3.9 g N-Acetyl-S-benzoyl.-D-cystein-4-nitrophenylester, erhalten analog J.Org.Chem. 27 (1962), Seite 3329, nach Beispiel 3 A mit 4,1 g L-1.2.3-4-Tetrahydro-isochinolin-3-carbonsäure-tert.-butylester-tosylat und 1.28 ml N-Ethylmorpholin um. Das Rohprodukt wird durch Filtration in chloroformischer Lösung über Silicagel gereinigt. Ausbeute 3,65 g (73 %) einer halbfesten Masse, die in der DC einheitlich ist und eine befriedigende Elementaranalyse zeigt.

Zur Abspaltung des tert.-Butylalkohols aus der Estergruppe bewahrt man die Verbindung 40 Minuten in Trifluoressigsäure auf, die sodann im Vakuum abdestilliert wird. Der Rückstand wird in 50 %igem, wäßrigen Methanol

gelöst und mit einem schwach basischen Ionenaustauscher in der Acetatform behandelt, bis der pH-Wert von etwa 2 auf etwa 3-4 zurückgegangen ist. Dann wird vom Austauscher abfiltriert. NachAbdestillieren des Lösungsmittels im Vakuum und Trocknen über $P_2O_5$ erhält man eine harte, pulverisierbare Masse. Ausbeute 2.5 g (81 %). Zur Analyse wird das Cyclohexylammonium- oder Dicyclohexylammoniumsalz aus Isopropanol umkristallisiert. Elementaranalyse der Salze korrekt.

Beispiel 5

N-Acetyl-D-cysteinyl-L-1.2.3.4-tetrahydroisochinolin-3-carbonsäure

1 g der nach Beispiel 4 erhaltenen Verbindung wird in 4 N methanolischem Ammoniak gelöst und 2 Stunden unter Stickstoff aufbewahrt. Aufarbeitung nach Beispiel 2. Ausbeute 0,54 g (71 %).

Beispiel 6

(2-Methyl-3-acetylmercaptopropionyl-L-1.2.3.4-tetrahydroisochinolin-3-carbonsäure

A. 2-Methyl-3-acetylmercaptopropionsäure-2.4.5-trichlorphenylester

Man stellt in bekannter Weise aus 16,2 g der Säure, 19.7 g 2.4.5-Trichlorphenol und 22 g Dicyclohexylcarbodiimid in Tetrahydrofuran den Trichlorphenylester her. Nach Filtration wird die Verbindung durch Chromatographie an Silicagel mit Tetrahydrofuran als Eluans gereinigt. Ausbeute 29.8 g (79 %). Schmp. 40-41°.

B. (2-Methyl-3-acetylmercaptopropionyl)-L-1.2.3.4-tetrahydroisochinolin-3-carbonsäure

Man stellt aus 1.77 g 1.2.3.4-Tetrahydro-isochinolin-3-carbonsäure und 1.05 g Tetramethylguanidin in 10 ml Methanol unter Stickstoff das entsprechende Salz her, destilliert das Methanol im Vakuum ab und löst den Rückstand unter Stickstoff in 10 ml Dimethylformamid.

Zu dieser Lösung gibt man 3.4 g des nach A erhalten 2-Methyl-3-acetylmercaptopropionsäure-aktivester und 0.1 g 1-Hydroxy-benzotriazol in 10 ml Dimethylformamid und bewahrt das ganze über Nacht bei Raumtemperatur auf. Nach Abdestillieren des Lösungsmittels reinigt man wie in Beispiel 1 E beschrieben und erhält 1.0 g der Titelverbindung, die mit der nach Beispiel 1 E erhaltenen Verbindung identisch ist.

Beispiel 7

(2-Methyl-3-nicotinoylmercaptopropionyl)-L-1.2.3.4-tetrahydro-isochinolin-3-carbonsäure

A. Nicotinsäure-N-hydroxysuccinimidester

Man stellt in bekannter Weise aus 30.8 g Nicotinsäure, 34.5 g N-Hydroxysuccinomid und 55 g Dicyclohexyl-carbodiimid in 800 ml Dimethylformamid die Titelverbindung her. Nach vollendeter Reaktion wird im Vakuum auf ein Drittel eingedampft, auf 0°C gekühlt, filtriert und das Filtrat im Vakuum zur Trockene gebracht. Der Rückstand wird aus 200 ml Isopropanol umkristallisiert. Ausbeute 49,0 g Schmp. 137-138 °C.

B. (2-Methyl-3-nicotinoylmercaptopropionyl-L-1.2.3.4-tetrahydro-isochinolin-3-carbonsäure

3,3 g des nach A erhaltenen Aktivesters werden mit 2,8 g N-(2-Methyl-3-mercaptopropionyl)-L-1.2.3.4-tetrahydro-isochinolin-3-carbonsäure und 1.28 ml N-Ethylmorpholin in 50 ml Dimethylformamid umgesetzt. Man destilliert nach Stehen über Nacht das Lösungsmittel im Vakuum ab, digeriert den Rückstand mit verdünnter Essigsäure und Wasser, trocknet ihn im Vakuum und nimmt ihn in wenig Isopropanol auf. Durch Zugabe von Cyclohexylamin wird das Cyclohexylammoniumsalz hergestellt, das zur Reinigung aus Isopropanol umkristallisiert wird. Die freie Verbindung wird durch Filtration der Lösung des Salzes in 50-prozentigem, wäßrigen Methanol über einen schwach sauren Ionenaustauscher erhalten. Man destilliert das

Lösungsmittel im Vakuum ab und erhält nach dem Trocknen des Rückstandes ein pulverisierbares Harz. UV-Extinktionskurve und Elementaranalyse weisen der Verbindung die korrekte Struktur zu.

Beispiel 8
N-(2-Methyl-3-mercaptopropionyl)-L-1.2.3.4-tetrahydro-isochinolin-3-carbonsäure

17.7 g 1.2.3.4-Tetrahydro-isochinolin-3-carbonsäure werden in bekannter Weise durch Salzsäure oder Thionylchlorid in Methanol in das Methylester-hydrochlorid überführt. Die Verbindung wird nach Abdestillieren des Lösungsmittels durch Digerieren mit Ether isoliert.

Den trockenen Rückstand setzt man nach Beispiel 1 D mit 16,2 g 2-Methyl-3-acetylmercaptopropionsäure, 12,8 ml N-Ethylmorpholin und 22 g Dicyclohexyl-carbodiimid in Methylenchlorid um. Aufarbeitung analog Beispiel 1 D.

Der ölige Rückstand wird in 150 ml Dioxan-Methanol 1:1 gelöst. Man gibt unter Rühren und unter Stickstoff 2N NaOH tropfenweise zu und hält den pH-Wert bei 12.5 - 13. Wenn keine Natronlauge mehr verbraucht wird, behandelt man die Lösung mit 50-prozentigem, wäßrigen Methanol und engt die vereinigten Lösungen im Vakuum ein. Der Rückstand wird mit Petrolether digeriert und im Vakuum getrocknet. Ausbeute 7.8 g. Die Verbindung ist in der Dünnschichtchromatographie mit der nach Beispiel 2 erhaltenen identisch.

Beispiel 9
ß,ß-Dithio-diisobutyryl-bis-L-1.2.3.4-tetrahydro-isochinolin-3-carbonsäure

A. 4 g L-1.2.3.4-Tetrahydro-isochinolin-3-carbonsäure-tert.-butylester-tosylat werden mit 1,2 g ß,ß-Dithiodiisobuttersäure, hergestellt nach Svensk kem. Tidskr. 55 (1943), Seite 170, und 1,1 g Dicyclohexylcarbodiimid in 50 ml Dimethylformamid in Anwesenheit

von 1,28 nl N-Ethylmorpholin umgesetzt. Das Reaktionsprodukt wird nach Filtration mit Wasser ausgefällt und getrocknet. Zur Spaltung des tert.-Butylesters behandelt man das Produkt 45 Minuten mit Trifluoressigsäure, die alsdann im Vakuum abdestilliert wird. Der Rückstand wird in 80 ml Methanol gelöst, die Lösung mit wenig schwach basischem Ionenaustauscher in der Acetatform verrührt und nach Filtration im Vakuum zur Trockene gebracht. Es entsteht eine harzige Masse, die nach längerem Stehen unter Ether/Petrolether fest wird und sich verreiben läßt. Sie wird vorteilhaft in ein Alkali- oder Ammoniumsalz überführt und ist dann besser zu handhaben.

B. 2 g der nach Beispiel 2 hergestellten Verbindung werden in einem Gemisch aus Ethanol und Natriumphosphatpuffer pH 6 gelöst. Man gibt unter Rühren tropfenweise Jod bis zur gleichbleibenden Gelbfärbung zu, entfärbt mit Thiosulfat und destilliert das Ethanol im Vakuum ab. Nun wird mit 4N Salzsäure vorsichtig bis pH 1.5 - 2 angesäuert und die Titelverbindung mit Essigester ausgeschüttelt. Die Essigesterlösung wird mit wenig Wasser gewaschen, über $Na_2SO_4$ getrocknet und im Vakuum eingedampft. Ausbeute 1,77 g. Zur Analyse wird in Acetonitril das Cyclohexylaminsalz hergestellt. Schmp. 186-190°C. Elementaranalyse korrekt.

Beispiel 10

3-Mercapto-2-methylpropanoyl-1.2.3.4-tetrahydrochinolin-2-carbonsäure

1.63 g 2-Methyl-3-acetylmercapto-propionsäure und 2.3 g 1.2.3.4-Tetrahydrochinolin-2-carbonsäuremethylesterhydrochlorid, hergestellt nach Ber. 61 (1928), Seite 2377, werden unter Stickstoff in 20 ml Dimethylacetamid gelöst. Man gibt ferner nacheinander 1.35 g 1-Hydroxy-benzotriazol, 1.28 ml N-Ethylmorpholin und 2.2 g Dicyclohexylcarbodiimid zu und rührt 4 Stunden bei Raumtemperatur. Dann filtriert

man und destilliert aus dem Filtrat das Lösungsmittel im Vakuum ab. Der Rückstand wird in 30 ml Methanol gelöst. Man rührt und tropft unter Stickstoff 10 ml 4N NaOH zu und versetzt 10 Minuten nach beendeter Zugabe mit 10 ml 4N HCl. Dann wird das Gemisch im Vakuum zur Trockene gebracht. Nach Abdestillieren des Lösungsmittels reinigt man durch Chromatographie an 130 g $SiO_2$ im System Chloroform/Isopropanol/Essigsäure (50:10:3). Ausbeute 2.3 g. Elementaranalyse und NMR-Spektrum korrekt.

Beispiel 11

2-Methyl-3-nicotinoylmercaptopropionyl-1.2.3.4-tetrahydrochinolin-2-carbonsäure

Man löst 1.4 g der nach Beispiel 10 erhaltenen Verbindung in 10 ml Dimethylformamid und läßt unter Stickstoff mit 1.1 g Nicotinsäure-N-hydroxysuccinimidester (hergestellt in üblicher Weise aus Nicotinsäure, N-Hydroxysuccinimid und Dicyclohexyl-carbodiimid, Schmelzpunkt 137 - 138°C) reagieren. Nach 3 Stunden wird das Lösungsmittel abdestilliert und der Rückstand analog Beispiel 10 chromatographiert. Ausbeute 0.7 g. UV- und NMR-Spektrum weisen das Produkt als die gesuchte Verbindung aus.

Beispiel 12

2-Methyl-3-acetylmercaptopropionyl-indolin-2-carbonsäure

Man stellt aus 2-Methyl-3-acetylmercaptopropionsäure mit 10 % Überschuß Thionylchlorid in Methylenchlorid durch dreistündiges Erwärmen das Säurechlorid her, das durch Destillation bei 1 mm Hg rein erhalten wird.
1.9 g des Säurechlorids werden mit dem Tetramethylenguanidiniumsalz von 1.63 g Indolin-2-carbonsäure in Dimethylacetamid unter Stickstoff umgesetzt. Man erhält nach Aufarbeitung und Reinigung analog Beispiel 10 2.9 g reine Titelverbindung mit korrekter Elementaranalyse.

Patentansprüche:

1. Verbindung der Formel I

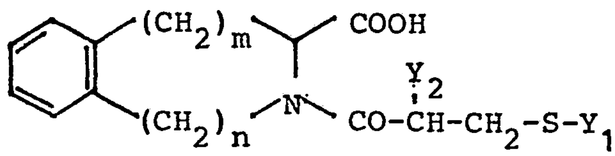

(I)

in welcher bedeuten:

n 0 oder 1,

m 1 oder 2, aber n + m ≦ 2

$Y_1$ Wasserstoff, Niederalkanoyl oder -cycloalkanoyl, Benzoyl, Nicotinoyl oder einen Rest der Formel II

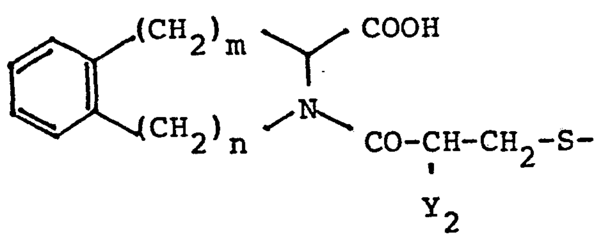

(II)

$Y_2$ Methyl, Amino, Niederalkanoylamido, Niedercycloalkaloylamido, Benzoylamido oder Nicotinoylamido sowie deren Salze.

2. Verfahren zur Herstellung einer Verbindung der Formel I, dadurch gekennzeichnet, daß man einen Niederalkylester einer Verbindung der Formel III

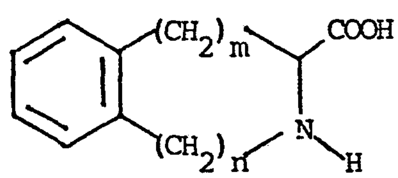

(III)

oder ein Salz dieser Verbindung mit einer starken, nicht flüchtigen organischen Base mit einem aktivierten Derivat einer Carbonsäure der Formel IV

$$HOOC-\overset{Y_2}{CH}-CH_2-S-Y_1$$

Patentansprüche für Österreich:

1. Verfahren zur Herstellung einer Verbindung der Formel I

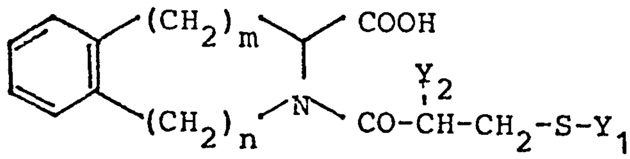

(I)

in welcher bedeuten:

n 0 oder 1,

m 1 oder 2, aber $n + m \leq 2$

$Y_1$ Wasserstoff, Niederalkanoyl oder -cycloalkanoyl, Benzoyl, Nicotinoyl oder einen Rest der Formel II

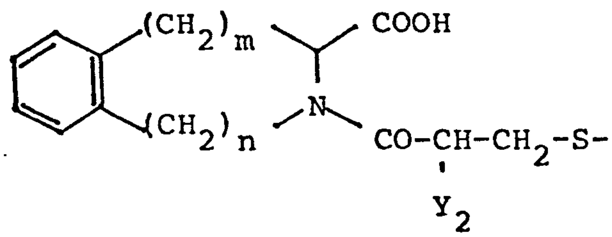

(II)

$Y_2$ Methyl, Amino, Niederalkanoylamido, Niedercyclo-alkaloylamido, Benzoylamido oder Nicotinoylamido

sowie deren Salzen,

dadurch gekennzeichnet, daß man einen Niederalkylester einer Verbindung der Formel III

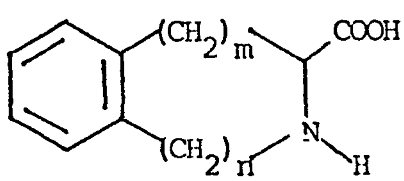

(III)

oder ein Salz dieser Verbindung mit einer starken, nicht flüchtigen organischen Base mit einem aktivierten Derivat einer Carbonsäure der Formel IV

$$HOOC-\overset{Y_2}{\overset{|}{C}}H-CH_2-S-Y_1$$

in der $Y_1 \neq H$ ist, umsetzt, aus dem erhaltenen Produkt die organische Base bzw. die Estergruppe abspaltet, gegebenenfalls einen in der Position $Y_1$ stehenden Säurerest abspaltet oder durch einen anderen austauscht und, falls die Verbindung eine freie SH-Gruppe hat, gewünschtenfalls das Produkt zur Disulfidverbindung oxidiert.

3. Verfahren nach Anspruch 2, dadurch gekennzeichnet, daß die organische Base mittels einer Säure oder eines stark sauren Ionenaustauscher abgespalten wird.

4. Verfahren nach Anspruch 2, dadurch gekennzeichnet, daß der in der Position $Y_1$ stehende Säurerest mit Alkali abgespalten wird.

5. Verwendung der Verbindungen gemäß Anspruch 1 zur Behandlung des Bluthochdrucks.

6. Verbindung gemäß Anspruch 1, in der m=n=1, $Y_1$ Wasserstoff und $Y_2$ Methyl ist.

in der $Y_1 \neq H$ ist, umsetzt, aus dem erhaltenen Produkt die organische Base bzw. die Estergruppe abspaltet, gegebenenfalls einen in der Position $Y_1$ stehenden Säurerest abspaltet oder durch einen anderen austauscht und, falls die Verbindung eine freie SH-Gruppe hat, gewünschtenfalls das Produkt zur Disulfidverbindung oxidiert.

2. Verfahren nach Anspruch 1, dadurch gekennzeichnet, daß die organische Base mittels einer Säure oder eines stark sauren Ionenaustauscher abgespalten wird.

3. Verfahren nach Anspruch 1, dadurch gekennzeichnet, daß der in der Position $Y_1$ stehende Säurerest mit Alkali abgespalten wird.

0029488

Europäisches Patentamt

**EUROPÄISCHER TEILRECHERCHENBERICHT,** der nach Regel 45 des Europäischen Patentübereinkommens für das weitere Verfahren als europäischer Recherchenbericht gilt

Nummer der Anmeldung
EP 80 10 5514

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| | DE - A - 2 703 828 (E.R. SQUIBB)<br>* Ansprüche 1,28,29; Seite 13, letzter Absatz; Seite 14, Absatz 1; Seite 21, letzter Absatz; Seite 22, Absatz 1 *<br>-- | 1,2 |
| P | EP - A - 0 012 845 (TANABE SEIYAKU CO. LTD.)<br>* Ansprüche *<br>-- | 1-4,6 |
| P | FR - A - 2 448 533 (MORTON-NORWICH PROD.)<br>* Ansprüche *<br>-- | 1-4,6 |
| E | EP - A - 0 018 104 (TAKEDA YAKUHIN)<br>* Ansprüche *<br>----- | 1-4,6 |

KLASSIFIKATION DER ANMELDUNG (Int. Cl.[3])

C 07 D 209/42
215/48
217/26
401/12
401/14
A 61 K 31/40
31/47

RECHERCHIERTE SACHGEBIETE (Int. Cl.[3])

C 07 D 207/00
209/00
215/00
217/00
401/00
A 61 K 31/00

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-4,6
Unvollständig recherchierte Patentansprüche:
Nicht recherchierte Patentansprüche: 5
Grund für die Beschränkung der Recherche:

Verfahren zur therapeutischen Behandlung des menschlichen oder tierischen Körpers (Siehe Art. 52(4) des Europäischen Patentübereinkommens).

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

| Recherchenort | Abschlußdatum der Recherche | Prüfer |
|---|---|---|
| WIEN | 12-11-1980 | ONDER |